# EUROPEAN PATENT SPECIFICATION

(11) **EP 0 785 791 B1**
(45) Date of publication and mention of the grant of the patent: **27.10.1999**
(21) Application number: 94911634.7
(22) Date of filing: 17.03.1994
(51) Int. Cl.: A61K 35/48, A61K 35/55

(54) **PROSTATE EXTRACT SUPPLEMENTED WITH ZINC**
MIT ZINK SUPPLEMENTIERTER PROSTATA-EXTRAKT
EXTRAIT PROSTATIQUE COMPLETE PAR DU ZINC

(43) Date of publication of application: 30.07.1997
(73) Proprietor: Song, Moon K., Northridge, CA 91326 (US)
(72) Inventor: Song, Moon K., Northridge, CA 91326 (US)
(74) Representative: Dörries, Hans, Dr.
(86) International application number: PCT/US94/02909
(87) International publication number: WO 95/24911

(56) References cited:
- EP-A- 0 071 357
- EP-A- 0 181 689
- EP-A- 0 372 676
- WO-A-93/00894
- SU-A- 1 740 004
- DIABETES RESEARCH, vol. 31, no. 4, 1996, pages 157-170, XP000612202 M.K. SONG ET AL: "ANIMAL PROSTATE EXTRACT AMELIORATES DIABETIC SYMPTOMS BY STIMULATING INTESTINAL ZINC ABSORPTION IN RATS"
- DATABASE WPI Week 9512 Derwent Publications Ltd., London, GB; AN 95-087893 XP002019789 & RU-A-1 417 244 (KHAVINSON V K) , 30 June 1994
- J.E.F. REYNOLDS (ED): "MARTINDALE, THE EXTRA PHARMACOPOEIA" 1989 , THE PHARMACEUTICAL PRESS , LONDON XP002019788 * page 1365, last line *
- J.Nutrition,vol.109(1979),pages 2152-2159
- Life Sciences,vol.42(1988),pages 687-694

## Description

The present invention relates to compositions and pharmaceutical preparations that contain zinc prostate extract supplemented with zinc

### BACKGROUND OF THE INVENTION

Diabetes is one of the most common metabolic disorders in humans. Indeed, nearly 1 million Americans are afflicted with juvenile-onset diabetes. This form of the disease is also known as insulin-dependent or Type I diabetes, and usually appears abruptly during childhood or young adulthood. Type II, or non-insulin-dependent diabetes is characterized by a less abrupt onset. Type II diabetes commonly occurs beyond the age of 40 or so. Both types of diabetes impair the body's ability to access blood glucose for use as an energy source. Chronically high levels of blood sugar gradually damage many tissues and organs of the body.

Substantial efforts have been directed toward understanding the causes and consequences of diabetes. A strong autoimmune component is now believed important in the etiology of type I diabetes (see *Science* **225**:1381 (1984), and Immunology Today 5:230 (1984)). The metabolic consequences of experimentally induced diabetes have also been explored. For example, in *Diabetes* **31**:426 (1982) Schneir et al. demonstrated that collagen, an important structural protein of skin, has a significantly decreased half-life in diabetic rats. It is well appreciated that both forms of diabetes impact the tissues of the body in a global fashion.

Despite the availability of insulin treatment, diabetes remains a serious disease that is responsible for many deaths and substantial morbidity worldwide. For example, the life-span of the average diabetic is shortened by as much as 50%. Although insulin treatment can assist in regulating blood sugar levels, the degree of this control is typically insufficient to prevent many of the sequelae from diabetes. The consequences from long term diabetes can include eye damage, often leading to blindness; circulatory problems; problems with wound healing; and other serious consequences. Improved treatments for diabetes are urgently required.

EP-A1-0 071 357 discloses a composition of an effective amount of (a) gamma-linolenic acid and/or dihomo-gamma-linolenic acid, optionally in association with linoleic or other fatty acids, said acids being used if desired as physiologically functional derivatives thereof, in conjunction with (b) an effective amount of one or more of natural or synthetic carotenoids or retinoids having, or giving rise in the body to compounds having, Vitamin A activity. The compositions are for use in therapy of a condition in which enhancement of 1-series prostaglandins is required.

### SUMMARY OF THE INVENTION

It is an object of the invention to provide a pharmaceutical composition for the treatment of diabetes comprising, in a form administrable to a mammal:
(a) a composition comprising an organic extract of animal prostatic tissue, said extract containing unsaturated fatty acids;
(b) at least one pharmaceutically acceptable excipient; and
(c) additionally zinc.

Another object of the invention is the said pharmaceutical composition wherein the unsaturated fatty acids comprise essential fatty acids selected from the group consisting of prostaglandins and prostaglandin precursors.

Still another object of the invention is the said pharmaceutical composition wherein the unsaturated fatty acids comprise essential fatty acids selected from the group consisting of prostaglandins and prostaglandin precursors, said pharmaceutical composition being in tablet or in capsule form, each tablet or capsule containing from 20 mg of zinc to 150 mg of zinc.

A further object of the invention is any of the aforesaid pharmaceutical compositions additionally comprising a protein hydrolyzate.

Another object of the invention is the said pharmaceutical composition additionally comprising a protein hydrolyzate wherein the protein hydrolyzate is in the form of amino acids or an incompletely hydrolyzed protein.

Yet another object of the invention is the said pharmaceutical composition comprising a protein hydrolysate wherein the composition contains unsaturated fatty acids, zinc sulfate and protein hydrolyzate in a ratio by weight of 10:1:5.

A further object of the invention is the said pharmaceutical composition comprising a protein hydrolyzate, optionally in the form of amino acids or incompletely hydrolyzed protein, in tablet or capsule form, each tablet or capsule containing about 200 mg prostate extract, about 20 mg of zinc and about 100 mg protein hydrolysate.

Another object of the invention is a pharmaceutical composition of any of claims 2 to 7 comprising zinc chelated with an extract of animal prostatic tissue obtainable by a process comprising
(a) mincing said tissue;
(b) resuspending the minced tissue in an aqueous solution;
(c) extracting from the resuspended tissue as a first group of molecules the saturated fatty acids with a first, nonpolar solvent which is as polar as, or less polar than, petroleum ether or hexane;
(d) discarding the fist solvent containing the saturated fatty acids;
(e) extracting as a second group of molecules the unsaturated fatty acids with a second solvent which is more polar than the first solvent; and
(f) removing the second solvent to obtain said extract.

A further object of the invention is the use of an organic extract of animal prostate tissue supplemented with zinc in the manufacture of a medicament for the treatment of diabetes.

### BRIEF DESCRIPTION OF THE FIGURES

Figure 1 shows the zinc concentrations of organ tissues of normal rats with and without prostate extract treatment. The open bars indicate without prostate extract and the cross-hatched bars indicates with prostate extract. In this figure, * indicates P < 0.05 and * * indicates P < 0.001.
Figure 2 shows zinc concentrations of organ tissues of diabetic rats with and without prostate extract treatment. The open bars indicate without prostate extract and the cross-hatched bars indicates with prostate extract. In this figure, * indicates P < 0.05 and ** indicates P < 0.001.

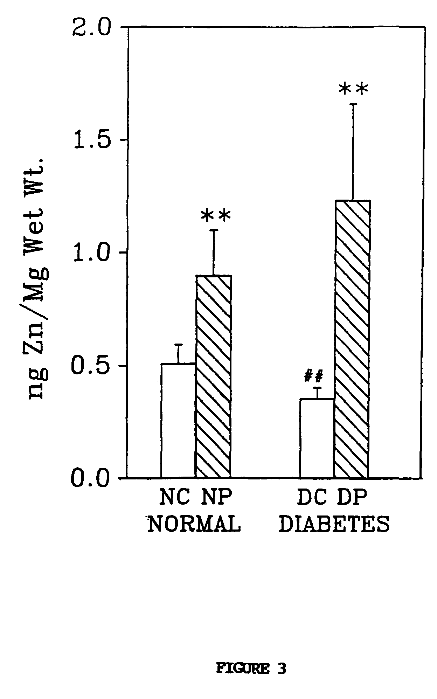

Figure 3 shows a comparison of zinc concentrations in organ tissues of normal and diabetic rats with and without prostate extract. NC refers to normal rats without prostate extract treatment. NP refers to normal rats with prostate extract treatment. DC refers to diabetic rats without protate extract treatment. DP refers to diabetic rats with prostate extract treatment. In this figure, * * indicates P < 0.001 when the values of protate extract treated rats are compared with those of untreated rates, and ## indicates P < 0.001 when the values of diabetic rats are compared with those of normal rats.

### DETAILED DESCRIPTION OF THE INVENTION

### Introduction

I have discovered that a composition of matter comprising a zinc chelated with extract of animal prostate tissue is useful for the treatment of diabetes. This composition may also be useful in treating other conditions that affect zinc and essential fatty acid metabolism.

For at least 60 years, the biomedical community has appreciated the fact that zinc metabolism is altered as a consequence of the diabetic condition. For example, in *J. Clin. Invest*. **17**:725(1938), Scott et al. showed that zinc is essential for insulin storage in the pancreatic B-cells of humans. In *Virchows Arch*. **B4**:94-302 (1970), Engelbart and Kief showed that acute stimulation of insulin secretion in rats reduced the zinc content in the B-cells of their pancreata. It is also well known that zinc participates in the storage of insulin, and that the amount of insulin stored during zinc deficiency is less than when zinc levels are normal. Thus, the important relationship between zinc metabolism and diabetes has been clearly established in the scientific literature.

An experimental finding presented under Example 1 confirmed that enhanced survival of diabetic test animals correlated with increased levels of dietary zinc. Based on this observation, I hypothesized that a dietary supplement which facilitated zinc utilization would also provide benefits to diabetic patients.

I considered prostaglandin precursors as possible diabetes therapeutic agents that could improve the symptoms associated with diabetes. My investigations concentrated on these substances because Song and Adham had previously demonstrated that at least some prostaglandins (PGE₂) can act as zinc-binding ligands (*J. Nutrition* **109**:2152 (1979)). The authors of this report also showed that other prostaglandins (PGA₂ and PGB₂) had no influence on zinc transport. Thus, only a subset of prostaglandins effectively cheated zinc, or otherwise influenced zinc transport. Additionally, in *Life Sciences* **42**:687 (1988), Song and Mooradian speculated that arachidonic acid, a prostaglandin precursor, played a role in the control of zinc flux across the intestinal epithelium of diabetic rats. Arachidonic acid is another example of a prostaglandin precursor.

Although it would seem desirable to alleviate diabetic symptoms by directly administering an appropriate prostaglandin, this approach is impractical. The fact that prostaglandins have very short half-lives prohibits their use as therapeutics.

Since prostaglandins are synthesized from unsaturated fatty acid precursors, I focused on these latter compounds as candidates for diabetes therapeutics. Furthermore, my search concentrated on sources of unsaturated fatty acids that could be administered as dietary supplements. Altogether, three different sources of unsaturated fatty acids were tested as diabetes therapies.

The results from side-by-side comparisons of arachidonic acid, evening primrose oil, and a novel extract of animal prostate tissue indicated marked differences between the effectiveness of these agents as diabetes therapeutics. Specifically, the extract of animal prostate tissue most effectively lowered blood glucose, and raised cytosolic tissue glucose concentrations in experimental rats.

Most significantly, my investigations demonstrated that encapsulated zinc chelated prostate extract lowered the blood glucose readings of diabetic humans. This finding confirmed the effectiveness of this novel composition as a viable diabetes therapy.

### I. Crystalline Zinc Chelated with Prostate Extract

One aspect of the present invention is zinc chelated with prostate extract; i.e., crystals in which negatively charged fatty acids that are present in an organic extract of prostate tissue are bound by positively charged zinc ions.

The organic extract of prostatic tissue contains unsaturated fatty acids that comprise essential fatty acids. The essential fatty acids preferably are selected from the group consisting of prostaglandins and prostaglandin precursors. These fatty acids can be obtained from the prostates of animals such as the cow, sheep, or goat. The extraction can be accomplished by resuspending the prostates in a buffered aqueous solution, extracting the saturated fatty acids with a highly nonpolar organic solvent such as petroleum ether or hexane, extracting unsaturated fatty acids with a more polar organic solvent such as ethyl acetate or chloroform, and then adding a zinc salt in a quantity sufficient to chelate the fatty acids or amino acids present.

### II. Pharmaceutical Compositions

Pharmaceutical compositions, according to the present invention, preferably contain: (1) an extract of animal prostate tissue, (2) a zinc salt such as zinc sulfate, (3) a protein hydrolysate, and (4) at least one pharmaceutically acceptable excipient.

The protein hydrolysate may be in the form of amino acids or incompletely hydrolyzed proteins such as proteoses, peptones, or other partially hydrolyzed proteins, such as albumin.

The pharmaceutical compositions prepared according to the present invention preferably contain essential fatty acids, zinc sulfate, and protein hydrolysate in a ratio of 10:1:5 by weight. The pharmaceutical compositions can be packaged in tablet or capsule form by procedures that are well known in the pharmaceutical arts. Preferably, each tablet or capsule contains about 200 mg of prostate extract, about 20 mg of zinc and about 100 mg of protein hydrolysate, in addition to the pharmaceutically acceptable excipient or excipients. Suitable excipients for tablets and capsules include inert diluents, such as calcium carbonate, sodium carbonate or bicarbonate, lactose, or calcium phosphate; or binding agents, such as starch, gelatin, or acacia; or lubricating agents, such as magnesium stearate, stearic acid, or talc. The coating of the capsules can be gelatin or a soluble polymer, as is well understood in the art. The tablets or capsules are suitable for oral administration.

The pharmaceutical compositions described herein are useful for the treatment of diabetes, hypertension, impotence, and other diseases in which zinc or prostaglandin metabolism is impaired. In particular, diabetes can be treated by administering the zinc cheated prostate extract of the present invention to a diabetic mammal in a quantity sufficient to reduce blood glucose concentration in the mammal. Typical doses for patients with diabetes or hypertension, stated as the quantity of zinc, are from about 80 mg to about 150 mg of zinc. These doses can be adjusted by one of ordinary skill in the art according to such factors as the weight, age, sex, and state of health of the patient, as well as according to the response to a particular dosage.

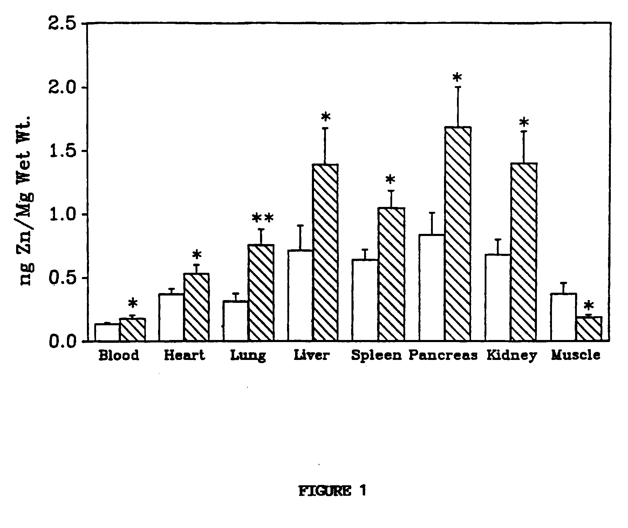

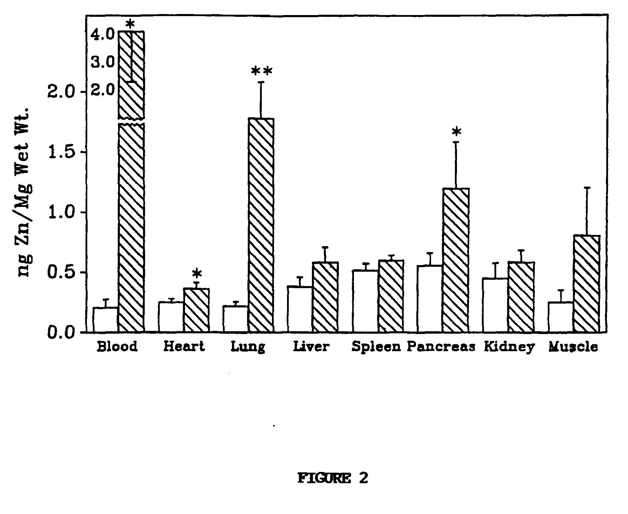

In the experiments illustrated in Figures 1 and 2, rats were anesthetized and then given 200 mg of prostate extract plus 200 µg zinc in 2 ml distilled water by intragastric intubation two hours before sacrifice. All the values are means ± SEM of six determinations. It can be seen that the prostate extract composition had a dramatic effect on zinc concentrations in all tissues tested in normal rats and on blood, heart, lung and pancreas of diabetic rats.

Figure 3 shows a comparison of zinc concentrations in organ tissues of normal and diabetic rats with and without prostate extract from the data shown in Figures 1 and 2. The mean values of tissue zinc concentrations in normal and diabetic rats treated with and without prostate extract were compared. It can be seen that there is a significant difference between normal and diabetic rats and also that prostate extract had a significant effect on both normal and diabetic rats.

The experiment described below in Example 1 illustrates a fundamental relationship between the level of dietary zinc consumption and longevity in diabetic rats. In this procedure, laboratory rats were made diabetic by injection with a chemical agent that is well known in the art. The diabetic rats were then fed diets that provided zinc in amounts that ranged from inadequate to excessive. The results from this simple experiment confirmed that dietary zinc influenced the life span of diabetic test animals.

### Example 1

### Effect of Dietary Zinc on the Survival of Diabetic Rats

Fifty-one rats were divided into three groups of 17 rats each. All of the rats were made diabetic by injection of streptozotocin (50 mg/kg). One week later the three groups of rats were fed diets having defined quantities of zinc. The first group was fed a zinc-deficient diet (1 µg Zn/g), the second group was fed a zinc-adequate diet (37.5 µg Zn/g), and the third group was fed a diet containing excess zinc (1 mg Zn/g). The number of surviving rats was counted after 25 days. The results presented in Table 1 summarize the survival of the test animals in this experiment.

**Table 1**

| THE SURVIVAL RATE OF DIABETIC RATS FED WITH DIFFERENT CONCENTRATIONS OF ZINC SURVIVAL RATES (No of Survived rats/No of diabetic rats) | | |
|---|---|---|
| (1 µg Zn/g) Zinc-Deficient | (37.5 µg Zn/g) Zinc-Adequate | (1 mg Zn/g) Zinc-Excess |
| 8/17 | 11/17 | 15/17 |

Example 2 describes the procedure that was used to isolate unsaturated fatty acids from animal prostate tissue. Prostate tissue contains zinc, prostaglandins, essential fatty acids, citric acid, testosterone and proteins. However, there is no evidence to indicate that an organic extract of prostate tissue is limited to these substances.

### Example 2

### Preparation of Crystalline Zinc Chelated Prostate Extract

Bovine prostates were obtained from a slaughterhouse, minced into small pieces and frozen at -70°C. The sliced tissue was suspended in a 10-fold excess (w/v) of 6.0 mM Tris-HCl buffer, pH 8.0, disrupted with a VIRTIS-45 homogenizer (Virtis Co., Gardner, New York), and centrifuged at 4°C for 20 minutes at 3,000 x g. The upper part of the fat was removed physically, and the supernatant was incubated at 37°C for one hour. The saturated fatty acids were extracted with petroleum ether. The remaining aqueous solution, which included unsaturated fatty acids, was acidified to pH 3.0 with 0.2 N HCl, and the unsaturated fatty acid mixture including prostaglandins was extracted 2 to 3 times with one volume each time of ethyl acetate mixed with isopropanol (1:1 v/v) or chloroform. The ethyl acetate or chloroform extracts were then combined. The unsaturated fatty acid solutions were either freeze-dried or evaporated to dryness under vacuum. The oil-like prostate extract that resulted from these procedures included unsaturated fatty acids. A preparation of zinc chelated unsaturated fatty acids was formed by mixing 200 mg of prostate extract with 20 mg zinc chloride and 100 mg of protein hydrolysate that was purchased from Sigma (St. Louis, MO).

Example 3 describes a procedure that was used to assess the effects of arachidonic acid, evening primrose oil and prostate extract on the symptoms of diabetic rats. Three sources of unsaturated fatty acids were tested for their relative abilities to improve the symptoms of diabetic rats. Arachidonic acid and evening primrose oil were two of the substances used in making this comparison. Evening primrose oil is known to be a rich source of both linoleic acid and linolenic acid. I also prepared and tested an organic extract of animal tissue as a source of unsaturated fatty acids that could positively influence diabetic symptoms in rats. All of the substances were administered as dietary supplements in conjunction with a dissolved zinc salt. My findings indicated the prostate extract most advantageously influenced the symptoms of diabetic rats.

### Example 3

### Investigation of the Effects of Unsaturated Fatty Acids on the Symptoms of Diabetic Rats

Rats were first made diabetic by injection with streptozotocin. These diabetic rats were then administered one of three unsaturated fatty acid preparations in their drinking water. The drinking water contained dissolved zinc sulfate together with either arachidonic acid, evening primrose oil or prostate extract that was prepared in accordance with the method of Example 2. The zinc sulfate solution was diluted to provide a final concentration of 10 mg/l of zinc. The arachidonic acid and prostate extract were each diluted to final concentrations of 100 mg/l. The evening primrose oil was diluted to a final concentration of 500 mg/l. The zinc and prostate extract concentrations used in this procedure were chosen to proportionally approximate a dosage that would be given to an adult human. In calculating the concentrations, I assumed that a 60 kg diabetic human would consume a daily dosage of 600-1000 mg of prostate extract and 60-100 mg zinc. This amounted to 10 mg of prostate extract and 1 mg of zinc per kg of body mass. The estimated daily water consumption for a normal rat was 40 ml. At 18 days post-streptozotocin injection, blood glucose measurements were recorded for all test animals using a GLUCOMETER ELITE (Miles Co.). The results of these glucose measurements are presented in Table 2.

**Table 2**

| BLOOD GLUCOSE CONCENTRATION (in mg/100 ml) IN DIABETIC RATS CHANGES AFTER CONSUMING WATER CONTAINING DIFFERENT CONSTITUENTS | | | |
|---|---|---|---|
| Experimental Conditions | Initial Values | Final Values | Imoroved Values |
| Normal Rats | 83.6 ± 2.6 | | |
| D-18d-Zn-PE | 495.0 ± 24.7 | 341.4 ± 17.4 | 153.6 ± 10.5 |
| D-18d-Zn-AA | 435.6 ± 24.7 | 353.4 ± 10.9 | 91.6 ± 24.4* |
| D-18d-Zn-EPO | 355 | 418 | -63 |
| D-18d-Zn only | 374.2 ± 37.6 | 377.0 ± 43.3 | 0.8 ± 24.3** |
| D-18d-DW | 321 | 359 | -48 |

| | | | |
|---|---|---|---|
| * P < 0.05 compared to the values of D-18d-Zn-PE | | | |
| * * P < 0.05 compared to the values of D-18d-Zn-PE | | | |
| D-18d = 18 days after induction of diabetes Zn-PE = Drinking water contained 10 mg Zn plus 100 mg of prostate extract/liter Zn-AA = Drinking water contained 10 mg Zn plus 100 mg of arachidonic acid/liter D-18d-EPO = Drinking water contained 8 mg Zn plus 500 mg of Evening Primrose oil/liter Zn-only = Drinking water contained 10 mg Zn/liter DW = Distilled water | | | |

The results shown in Table 2 confirmed that diabetic animals had significantly higher blood glucose levels than normal rats. Administration of zinc alone to diabetic rats did not effect blood glucose levels. Dietary administration of zinc together with evening primrose oil was also ineffective in reducing the blood glucose concentration of the diabetic animals. The combination treatment of zinc and arachidonic acid moderately reduced blood glucose concentrations. Surprisingly, the reduction of blood glucose that followed administration of zinc and prostate extract was clearly more pronounced than for any other treatment.

That prostate extract should efficiently reduce the blood glucose concentration of diabetic animals while evening primrose oil and arachidonic acid were ineffective or moderately effective, was an unexpected result. Significantly, this finding demonstrated that all sources of unsaturated fatty acids are not equal in their capacities to improve diabetic symptoms.

To independently assess a second indicator of the diabetic condition, cytosolic glucose concentrations were determined for various tissues in diabetic rats that had undergone various dietary supplementations. Example 4 presents the results of such determinations that were made using rats treated exactly as described under Example 3.

### Example 4

### Cytosolic Glucose Concentrations in Various Tissues of Diabetic Rats

Immediately following sacrifice of the experimental animals from Example 3 by injection of nembutal (50 mg/kg), various tissues were isolated and stored at -70°C. Each tissue was disrupted in a polytron homogenizer and then centrifuged at 2,000 x g for 20 minutes. The supernatants were either stored frozen or immediately used for determining glucose and protein concentrations. Glucose concentrations were measured using a model A23 glucose analyzer that was purchased from Yellow Springs Instruments Co. (Yellow Springs Ohio). Protein concentrations were determined according to the method described by Lowry et al. in *J. Biol. Chem.* **193**:265 (1951). The values obtained for the glucose readings were divided by the protein concentration readings to normalize the measurements. This normalization was necessary to control for the variable masses and protein yields that distinguished batches of different tissues. The results are shown in Table 3.

**Table 3**

| GLUCOSE CONCENTRATIONS OF DIFFERENT ORGAN CELL CYTOSOLS OF DIABETIC RATS FED DIFFERENT DIETS | | | | | |
|---|---|---|---|---|---|
| | Glucose Concentration (mg glucose/mg protein) | | | | |
| Organs | Zinc plus PE | Zinc plus AA | Zinc Only | Zinc plus EPO | Dist. Water |
| Heart | 184.3 ± 19.6 | 151.8 ± 14.0 | 111.9 ± 15.8* | 86.4 | 97.0 |
| Lung | 64.3 ± 9.5 | 59.3 ± 6.3 | 57.9 ± 10.3 | 56.8 | 51.0 |
| Liver | 181.8 ± 26.4 | 149.7 ± 22.0* | 122.1 ± 17.5* | 123.1 | 124.4 |
| Pancreas | 152.8 ± 11.1 | 126.0 ± 6.4# | 128.4 ± 12.2# | 89.6 | 112.2 |
| Spleen | 131.0 ± 3.6 | 103.1 ± 7.4** | 123.2 ± 9.1 | 123.1 | 124.4 |
| Kidney | 200.8 ± 15.4 | 157.5 ± 12.7# | 150.8 ± 11.5* | 119.3 | 118.9 |
| Muscle | 638.5 ± 67.0 | 398.5 ± 41.2** | 383.0 ± 43.7** | 317.7 | 338.5 |
| Intest. | 970.2 ± 74.0 | 685.6 ± 90.1* | 792.8 ± 94.3# | 605.4 | 702.2 |
| Total Ave. | 315.5 ± 112.2 | 228.9 ± 74.4* | 233.8 ± 86.9* | 190.2 ± 65.6 | 208.6 ± 76.7 |

| | | | | | |
|---|---|---|---|---|---|
| # Significant at P = 0.05 when compared the values of those rats given water containing prostate extract (PE) plus zinc. | | | | | |
| * Significant at P < 0.05 when compared the values of those rats given water containing PE plus zinc. | | | | | |
| ** Significant at P < 0.01 when compared the values of those rats given water containing PE plus zinc. | | | | | |

The results in Table 3 indicated a correlation between dietary supplementation with prostate extract and enhanced glucose uptake at the cellular level. Whereas diabetic tissues typically had low levels of free glucose, my results showed this level was dramatically increased in test animals that received the combination of prostate extract and zinc. Dietary administration of either arachidonic acid or evening primrose oil in combination with zinc had no effect on cytosolic glucose concentrations. The next step in the development of my invention was to extend the use of zinc chelated prostate extract beyond the treatment of laboratory animals.

The effectiveness of prostate extract in alleviating the symptoms of diabetic humans was also investigated. Example 5 describes changes in blood glucose concentrations that were recorded for diabetic volunteers after dietary supplementation with capsules that contained zinc chelated prostate extract.

### Example 5

### Effect of Dietary Administration of Prostate Extract on Blood Glucose levels of Diabetic Humans

Sixteen diabetic individuals were identified and recruited as volunteers to test the effects of dietary supplementation with zinc chelated prostate extract. Initial blood glucose readings were taken for all individuals using a GLUCOMETER ELITE (Miles Co.). All 16 volunteers then consumed encapsulated zinc chelated prostate extract 2-4 times each day for a period of 1 to 3 months. Each capsule contained 200 mg of prostate extract, 20 mg of zinc and gelatin. These capsules were prepared by Banner Pharmacap Co. (Chatsworth, CA) using zinc chelated prostate extract prepared according to the method detailed under Example 2. At the end of the test period, blood glucose measurements were repeated for all subjects. Table 4 summarizes the results of all blood glucose readings in this experiment.

**Table 4**

| BLOOD GLUCOSE CONCENTRATIONS OF MILD DIABETIC PATIENTS AFTER THE INTAKE OF BOVINE PROSTATE EXTRACT SUPPLEMENTED WITH ZINC CAPSULE | | | | |
|---|---|---|---|---|
| Patient Initials | Patient Age | Patient Sex | Range of Glucose Concentration Before Zinc Intake | Range of Glucose Concentration After Zinc Intake |
| JSS | 56 | F | 190-190 mg/100 ml | 90-135 mg/100 ml |
| KIM | 76 | F | 270-370 mg/100 ml | 95-179 mg/100 ml |
| SHK | 63 | M | 180-280 mg/100 ml | 120-180 mg/100 ml |
| DHS | 61 | F | 180-280 mg/100 ml | 130-170 mg/100 ml |
| HGL | 59 | M | 150-240 mg/100 ml | 100-160 mg/100 ml |
| HKK | 57 | M | 180-280 mg/100 ml | 130-180 mg/100 ml |
| SBS | 61 | M | 200-290 mg/100 ml | 130-200 mg/100 ml |
| SES | 62 | M | 180-250 mg/100 ml | 130-210 mg/100 ml |
| CLC | 64 | M | 150-260 mg/100 ml | 90-160 mg/100 ml |
| JJK | 49 | F | 280-320 mg/100 ml | 180-240 mg/100 ml |
| BSK | 59 | M | 210-350 mg/100 ml | 150-210 mg/100 ml |
| JAK | 51 | M | 160-250 mg/100 ml | 100-190 mg/100 ml |
| KUK | 59 | M | 180-260 mg/100 ml | 140-210 mg/100 ml |
| ESL | 58 | F | 150-240 mg/100 ml | 110-180 mg/100 ml |
| PHL | 62 | M | 180-250 mg/100 ml | 110-160 mg/100 ml |
| HSC | 78 | F | 210-310 mg/100 ml | 150-210 mg/100 ml |
| The Means ± SEM of mid points of each value: | | | | |
| | | | 236.56 ± 9.19 | 152.43 ± 6.88 |
| Paired t statistics of the two med-points of each value is P < 0.0001. | | | | |

Glucose clearance rates were also measured for diabetic patients before and after a regimen of dietary administration with zinc chelated prostate extract. In this experiment, I measured the rate at which a defined amount of glucose is removed from the blood stream of diabetic individuals. Whereas non-diabetics typically exhibit rapid glucose clearance rates, diabetic individuals exhibit lower rates. These lower rates of glucose clearance result from the diminished insulin activity that characterizes the diabetic condition. An increased rate of glucose clearance that followed administration of zinc chelated prostate extract indicates a therapeutic effect.

The advantage of using blood glucose clearance rate as an indicator of efficacy is that this test is not influenced by temporal changes of diet or exercise whereas blood glucose levels are effected. For this reason, comparison of blood glucose clearance rates before and after a regimen of dietary administration of zinc chelated prostate extract is believed to most accurately measure the positive effects of the therapeutic regimen.

Example 6 details the procedure used to measure the effect of dietary administration of zinc chelated prostate extract on the glucose clearance rates of diabetic volunteers.

### Example 6

### The Effect of Zinc Chelated Prostate Extract on the Glucose Clearance Rates of Diabetic Patients

Four diabetic volunteers were first identified at the Sepulveda V.A. Medical Center. After stabilizing their carbohydrate intakes at a level of 150-200 grams/day for a period of 3 days, glucose clearance rates were determined after drinking a 225 ml volume of solution that contained 75 grams of glucose. Blood glucose measurements were then taken at 0, 30, 60, 90, 120, 150 and 180 minutes after consuming the glucose solution. Blood glucose measurements were made using a ONE-TOUCH II GLUCOMETER (Lifescan, Inc., Milpitas, CA). The clearance rate was determined as the rate at which the glucose concentration decreased after having reached a maximal concentration.

After establishing the initial blood glucose clearance rates, three randomly selected volunteers consumed 4 capsules of zinc chelated prostate extract each day for a period of 3 months. The composition of the capsules used in this procedure was exactly as described under Example 5. The fourth diabetic volunteer was given a placebo. At the end of the 3 month trial period, blood glucose clearance rates were again determined for all volunteers. The quantitative results of this procedure are presented in Table 5.

**Table 5**

| GLUCOSE TOLERANCE TEST | | | | | |
|---|---|---|---|---|---|
| | | (mg Glucose/100 ml blood samples) | | | |
| Patients # | Gel Capsules | Initial GCR | Final GCR | Difference | % of Change |
| 1. | Test Capsules | 11 | 32 | 21 | 190.9 |
| 2. | Test Capsules | 57 | 75 | 18 | 31.6 |
| 3. | Test Capsules | 48 | 59 | 11 | 22.9 |
| 4. | Placebo | 102 | 95 | -7 | -6.8 |
| 5. | Placebo | 21 | 20 | -1 | -4.8 |

The results presented in Table 5 indicated the glucose clearance rates of all diabetic volunteers were substantially improved after a 3 month period of consuming zinc chelated prostate extract. A patient who received a placebo treatment showed no such improvement. Because the patient that received the placebo was only mildly diabetic, his initial glucose clearance rate was more nearly normal than any of the other volunteers.

Interestingly, the patient receiving the placebo in this test exhibited a decreased glucose clearance rate over the period of the experiment. This represents an ordinary trend for such a patient, since the glucose clearance rate becomes progressively lower as the disease becomes progressively more severe.

The findings presented here indicated that dietary supplementation with zinc chelated prostate extract improved the symptoms associated with diabetes. Although the mechanism by which prostate extract leads to improved symptoms in diabetic animals and man is not clearly understood, the powerful effects of prostate extract on the increased intestinal zinc absorption may be contributory. Regardless of the mechanism, I have demonstrated that dietary supplementation with prostate extract produces superior results with regard to improving diabetic symptoms when compared to regimens that include dietary administration with zinc, either alone or in combination with other examples of unsaturated fatty acids.

The present invention provides compositions that are convenient sources of both zinc and essential fatty acids. This composition can be used as a dietary supplement or treatment for diabetes or other conditions. Because the metabolism of zinc and the metabolism of essential fatty acids, including prostaglandin precursors, are interlinked, the use of such compositions is more effective than is the use of either zinc or fatty acids alone in treating diabetes.

## Claims

1. A pharmaceutical composition for the treatment of diabetes comprising, in a form administrable to a mammal:
(a) a composition comprising an organic extract of animal prostatic tissue, said extract containing unsaturated fatty acids;
(b) at least one pharmaceutically acceptable excipient; and
(c) additionally zinc.

2. The pharmaceutical composition of claim 1 wherein the unsaturated fatty acids comprise essential fatty acids selected from the group consisting of prostaglandins and prostaglandin precursors.

3. The pharmaceutical composition of claim 2 in tablet or in capsule form, each tablet or capsule containing from 20 mg of zinc to 150 mg of zinc.

4. The pharmaceutical composition of any of claims 1 to 3 additionally comprising a protein hydrolyzate.

5. The pharmaceutical composition of claim 4, wherein the protein hydrolyzate is in the form of amino acids or an incompletely hydrolyzed protein.

6. The pharmaceutical composition of claim 4 or 5, wherein the composition contains fatty acids, zinc sulfate and protein hydrolyzate in a ratio by weight of 10:1:5.

7. The pharmaceutical composition of claims 4 or 5, wherein the pharmaceutical composition is in tablet or capsule form, each tablet or capsule containing about 200 mg prostate extract, about 20 mg of zinc and about 100 mg protein hydrolysate.

8. The pharmaceutical composition of any of claims 2 to 7 comprising zinc chelated with an extract of animal prostatic tissue obtainable by a process comprising
(a) mincing said tissue;
(b) resuspending the minced tissue in an aqueous solution;
(c) extracting from the resuspended tissue as a first group of molecules the saturated fatty acids with a first, nonpolar solvent which is as polar as, or less polar than, petroleum ether or hexane;
(d) discarding the first solvent containing the saturated fatty acids;
(e) extracting as a second group of molecules the unsaturated fatty acids with a second solvent which is more polar than the first solvent; and
(f) removing the second solvent to obtain said extract.

9. Use of an organic extract of animal prostate tissue supplemented with zinc in the manufacture of a medicament for the treatment of diabetes.

## Patentansprüche

1. Pharmazeutische Zubereitung für die Behandlung von Diabetes, enthaltend in einer an Säugetiere verabreichbaren Form:
(a) ein Stoffgemisch, welches einen organischen Extrakt von tierischem Prostatagewebe enthält, wobei dieser Extrakt ungesättigte Fettsäuren enthält;
(b) mindestens einen pharmazeutisch akzeptablen Trägerstoff; und
(c) zusätzlich Zink.

2. Pharmazeutische Zubereitung nach Anspruch 1, wobei die ungesättigten Fettsäuren essentielle Fettsäuren umfassen, die ausgewählt sind aus der Gruppe, welche aus Prostaglandinen und Vorläuferstoffen für Prostaglandine besteht.

3. Pharmazeutische Zubereitung nach Anspruch 2 in Form von Tabletten oder Kapseln, wobei jede Tablette oder Kapsel 20 mg Zink bis 150 mg Zink enthält.

4. Pharmazeutische Zubereitung nach einem der Ansprüche 1 bis 3, welche zusätzlich ein Proteinhydrolysat enthält.

5. Pharmazeutische Zubereitung nach Anspruch 4, wobei das Proteinhydrolysat in Form von Aminosäuren oder eines unvollständig hydrolysierten Proteins vorliegt.

6. Pharmazeutische Zubereitung nach Anspruch 4 oder 5, wobei die Zubereitung Fettsäuren, Zinksulfat und Proteinhydrolysat in einem Gewichtsverhältnis von 10:1:5 enthält.

7. Pharmazeutische Zubereitung nach Anspruch 4 oder 5, wobei die pharmazeutische Zubereitung in Form von Tabletten oder Kapseln vorliegt und jede Tablette oder Kapsel etwa 200 mg Prostataextrakt, etwa 20 mg Zink und etwa 100 mg Proteinhydrolysat enthält.

8. Pharmazeutische Zubereitung nach einem der Ansprüche 1 bis 7, die Zink enthält, das mit einem Extrakt aus tierischem Prostatagewebe chelatisiert ist, wobei die Zubereitung erhältlich ist nach einem Verfahren, bei dem man
(a) das Gewebe zerkleinert;
(b) das zerkleinerte Gewebe in einer wäßrigen Lösung resuspendiert;
(c) aus dem resuspendierten Gewebe mit einem ersten, unpolaren Lösemittel, das so polar ist wie, oder weniger polar ist als, Petrolether oder Hexan als eine erste Gruppe von Molekülen die gesättigten Fettsäuren extrahiert;
(d) das erste Lösemittel, das die gesättigten Fettsäuren enthält, verwirft;
(e) als eine zweite Gruppe von Molekülen die ungesättigten Fettsäuren mit einem Lösemittel extrahiert, das stärker polar als das erste Lösemittel ist; und
(f) das zweite Lösemittel entfernt, um den Extrakt zu gewinnen.

9. Verwendung eines organischen Extrakts von tierischem Prostatagewebe, der einen Zusatz von Zink enthält, bei der Herstellung eines Arzneimittels zur Behandlung von Diabetes.

## Revendications

1. Composition pharmaceutique pour le traitement de diabètes comprenant, sous une forme administrable à un mammifère :
(a) une composition comprenant un extrait organique de tissu prostatique animal, ledit extrait contenant des acides gras insaturés;
(b) au moins un excipient pharmaceutiquement acceptable; et
(c) additionnellement du Zinc.

2. Composition pharmaceutique suivant la revendication 1, dans laquelle les acides gras insaturés comprennent des acides gras essentiels choisis dans le groupe comprenant les prostaglandines et les précurseurs de prostaglandines.

3. Composition pharmaceutique suivant la revendication 2, sous forme de comprimé ou de capsule, chaque comprimé ou capsule contenant de 20 mg de zinc à 150 mg de zinc.

4. Composition pharmaceutique suivant l'une quelconque des revendications 1 à 3, comprenant de plus un hydrolysat protéique.

5. Composition pharmaceutique suivant la revendication 4, dans laquelle l'hydrolysat protéique est sous la forme d'acides aminés ou d'une protéine incomplètement hydrolysée.

6. Composition pharmaceutique suivant l'une ou l'autre des revendications 4 et 5, dans laquelle la composition contient des acides gras, du sulfate de zinc et de l'hydrolysat protéique dans un rapport en poids de 10/1/5.

7. Composition pharmaceutique suivant l'une ou l'autre des revendications 4 et 5, dans laquelle la composition pharmaceutique est sous la forme de comprimé ou de capsule, chaque comprimé ou capsule contenant environ 200 mg d'extrait prostatique, environ 20 mg de zinc et environ 100 mg d'hydrolysat protéique.

8. Composition pharmaceutique suivant l'une quelconque des revendications 2 à 7, comprenant du zinc chélaté avec un extrait de tissu prostatique animal obtenable par un procédé comprenant :
(a) le hachage dudit tissu;
(b) la remise en suspension du tissu haché dans une solution aqueuse;
(c) l'extraction du tissu remis en suspension comme premier groupe de molécules des acides gras saturés avec un premier solvant, non polaire, qui est aussi polaire ou moins polaire que l'éther de pétrole ou l'hexane;
(d) l'écartement du premier solvant contenant les acides gras saturés;
(e) l'extraction comme second groupe de molécules des acides gras insaturés avec un second solvant qui est plus polaire que le premier solvant; et
(f) la séparation du second solvant pour obtenir l'extrait précité.

9. Utilisation d'un extrait organique de tissu prostatique animal complété avec du zinc dans la fabrication d'un médicament pour le traitement de diabètes.
